# EUROPEAN PATENT SPECIFICATION

(11) **EP 3 037 919 B1**
(45) Date of publication and mention of the grant of the patent: **07.07.2021**
(21) Application number: 14838613.9
(22) Date of filing: 08.05.2014
(51) Int. Cl.: G06F 3/01, G06F 9/44, G06F 13/14, A61B 5/22, G06F 3/0346, G06F 1/16, A61B 5/0488, A61B 5/11, A61B 5/00

(54) **WEARABLE BIOSIGNAL INTERFACE AND METHOD OF OPERATING WEARABLE BIOSIGNAL INTERFACE**
TRAGBARE BIOSIGNALSCHNITTSTELLE SOWIE VERFAHREN ZUM BETREIBEN DER TRAGBAREN BIOSIGNALSCHNITTSTELLE
INTERFACE DE BIOSIGNAL VESTIMENTAIRE ET MÉTHODE DE FONCTIONNEMENT D'INTERFACE DE BIOSIGNAL VESTIMENTAIRE

(30) Priority: 20.08.2013 KR 20130098476
(43) Date of publication of application: 29.06.2016
(73) Proprietor: Samsung Electronics Co., Ltd., Suwon-si, Gyeonggi-do 443-742 (KR)
(72) Inventor: CHOI, Chang Mok, Suwon-si Gyeonggi-do 443-803 (KR); KIM, Sang Joon, Suwon-si Gyeonggi-do 443-803 (KR); YOON, Seung Keun, Suwon-si Gyeonggi-do 443-803 (KR)
(74) Representative: Grünecker Patent- und Rechtsanwälte PartG mbB
(86) International application number: PCT/KR2014/004073
(87) International publication number: WO 2015/026047

(56) References cited:
- WO-A2-2008/073801
- KR-A- 20090 027 390
- KR-A- 20090 032 537
- KR-A- 20110 123 708
- KR-A- 20120 111 030
- US-A1- 2006 281 979
- US-A1- 2012 184 826
- None

## Description

### Technical Field

The following description relates to a wearable biosignal interface and an operation method of the wearable biosignal interface.

### Background Art

Recently, a new wearable type of mobile device has been emerging at an accelerated rate amid the proliferation of smartphones. Such a type of mobile device may display information obtained by a smartphone through a wireless data network, on a separate screen, by processing data without directly viewing the smartphone.

Generally, a gesture recognized by an accelerometer or a biosignal sensor may be used to operate the wearable mobile device. However, operating the wearable mobile device based on the gesture only may increase a probability of misrecognizing an intention of a user for operation. For example, in a case of the wearable mobile device operated by recognizing a hand gesture, even a gesture made by a severe movement of an arm connected to the hand may be recognized as the intention of a user for operation. One recently known device in the art is presented in WO 2008/073801 A2 under the title "Inertial Sensor Input Device" that describes a head motion input device for providing control to a computer. The device comprises a bio sensor and suppresses a signal if the user movement velocity is outside a range.

Another prior art device is known from KR 2012 0111030 A addressing a wearable sensor set and corresponding operating method of the same. This document teaches only using the bio sensor signal if the user is not moving.

### Disclosure of Invention

### Technical Solutions

Accordingly, there is a desire for a wearable mobile device model that may recognize an intention of a user for more precise operation.

The invention is defined by the independent claims. A preferred embodiment is disclosed in the dependent claim 2.

### Brief Description of Drawings

FIG. 1 is a diagram illustrating an example of a configuration of a wearable biosignal interface according to present invention.
FIGS. 2 through 5 are diagrams illustrating examples of determining a valid second signal based on a first signal and a second signal according to present invention.
FIG. 6 is a diagram illustrating an example of performing a command given to an external device, using a magnitude of a first signal and a magnitude of a second signal.
FIG. 7 is a flowchart illustrating an example of an operation method of a wearable biosignal interface according to present invention.

### Best Mode for Carrying Out the Invention

Reference will now be made in detail to embodiments of the present invention, examples of which are illustrated in the accompanying drawings, wherein like reference numerals refer to the like elements throughout. The embodiments are described below in order to explain the present invention by referring to the figures. The subject matter of present invention is provided in the independent claims, preferred embodiments are defined in the dependent claims.

As described herein, a sensor refers to a sensor or a plurality of sensors that are attached to a target to be in contact therewith so as to obtain a signal generated from the target or detect a motion of the target.

For example, the sensor includes a biosensor that is attached to a body part of a living object, for example, a wrist or a forearm, to obtain a signal generated by muscle contraction or muscle relaxation of the body part. The sensor described above will be referred to as a biosignal sensor in this description. The biosignal sensor detects biological information from a target and converts the information into a signal waveform to generate a biosignal. The biosignal sensor may include a photo sensor, a piezoelectric sensor, a force sensor, an electromyogram (EMG) sensor, and the like.

For example, the sensor may include an acceleration sensor, a gyro sensor, a geomagnetic sensor, and a Global Positioning System (GPS) sensor, which may directly or indirectly monitor a motion performed by the living object and obtain a signal associated with the motion performed by the living object. Hereinafter, the sensor described above will be referred to as a motion sensor.

In a case of controlling an external device using a biosignal obtained by the biosignal sensor, a wearable biosignal interface according to an embodiment ignores the biosignal when a magnitude of a signal associated with a motion of a target exceeds a threshold and conversely, receive the biosignal when the magnitude of the signal associated with the motion of the target is less than or equal to the threshold.

The wearable biosignal interface perceives a motion unintentionally or spontaneously performed by a user, and ignores a biosignal obtained as a result of such a motion. Accordingly, accuracy of determining, based on the biosignal, an intention of a user for operating a device may be improved.

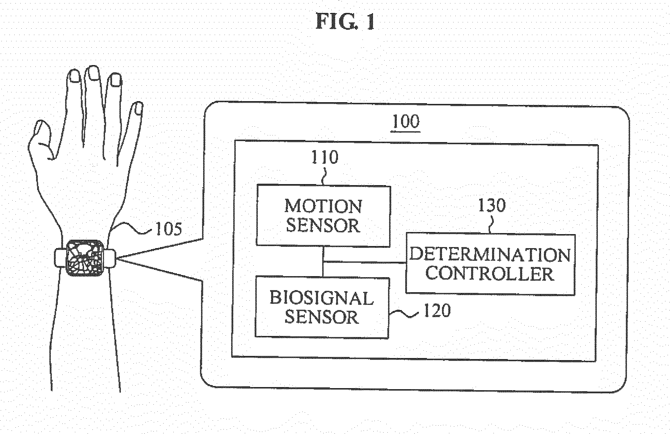

FIG. 1 is a diagram illustrating an example of a wearable biosignal interface 100 according to an embodiment of the present invention.

Referring to FIG. 1, the wearable biosignal interface 100 includes a motion sensor 110, a biosignal sensor 120, and a determination controller 130.

The motion sensor 110 detects a motion of a target 105 and obtains a first signal. Here, the first signal may include an acceleration signal, a rotation signal, a bearing signal, and a coordinate signal, which are associated with velocity variance based on a motion status of the target 105.

The motion sensor 110 monitors a motion of the target 105 and receives the first signal recognizing a movement associated with the motion. Also, the motion sensor 110 identifies a gesture to be performed by the target 105 when a second signal is obtained and obtains the first signal associated with the identified gesture.

For example, the motion sensor 110 may include an acceleration sensor. In this case, the motion sensor 110 converts an acceleration signal corresponding to the obtained first signal into a velocity component, through calculating an integral. The motion sensor 110 numerically indicates a magnitude of a motion of the target 105.

When the target 105 is an arm which is flexed in any direction, the motion sensor 110 obtains a first signal associated an "arm motion" based on the flexing. Also, the motion sensor 110 measures a magnitude of the obtained first signal and measures a magnitude of a velocity of the "arm motion."

The motion sensor 110 may include a gyrosensor. In this case, the motion sensor 110 detects, using the gyrosensor, a rotation signal corresponding to the first signal.

When the target 105 is an arm performing a large rotation, the motion sensor 110 obtains a first signal associated with an "arm rotation" based on the rotating performed by the arm. Also, the motion sensor 110 measures a magnitude of the obtained first signal and measures a magnitude of a rotational velocity of the " arm rotation."

The motion sensor 110 may include a geomagnetic sensor or a GPS sensor. In this case, the motion sensor 110 detects, using the geomagnetic sensor or the GPS sensor, a bearing signal or a coordinate signal corresponding to the first signal.

The motion sensor 110 is fixed to a form of a bracelet fastened onto the target 105, along with the biosignal sensor 120.

The biosignal sensor 120 is in contact with the target 105 and obtains a second signal from the target 105. Here, the second signal is a biosignal associated with biological information, for example, muscle contraction and muscle relaxation occurring in the target 105. The second signal may be a bio-electric/magnetic signal, a bio-impedance signal, and a bio-mechanical signal generated in association with a muscle.

The biosignal sensor 120 is in contact with the target 105 and obtains the second signal generated by at least one of muscle contraction and muscle relaxation. When the target 105 is a wrist, the biosignal sensor 120 detects the second signal generated by muscle contraction or muscle relaxation of the wrist.

Here, the target 105 may be a wrist, a forearm, a face, a neck, and the like of a living object, in which a muscle generating the second signal is positioned.

The biosignal sensor 120 includes at least one biosensor that detects the second signal from a muscle of the target 105, and monitors contraction or relaxation of the muscle using the at least one biosensor. The biosensor may be a photo sensor, a piezoelelctric sensor, a force sensor, an electromyogram (EMG) sensor, and the like.

For example, the biosignal sensor 120 may include the photo sensor. In this case, the biosignal sensor 120 obtains, using the photo sensor, the second signal associated with a status of the target 105, based on light scattering.

The biosignal sensor 120 may include the piezoelelctric sensor or the force sensor. In this case, the biosignal sensor 120 obtains, using the piezoelelctric sensor or the force sensor, the second signal associated with an extension and contraction status of the target 105.

The biosignal sensor 120 may include the EMG sensor. In this case, the biosignal sensor obtains, using the EMG sensor, the second signal associated with muscle contraction or relaxation of the target 105.

The biosignal sensor 120 may be fixed to the bracelet fastened onto the target 105, along with the motion sensor 110. Here, a sensing surface of the biosignal sensor 120 is fixed to the bracelet to be in a direct contact with the target 105. On the sensing surface, the at least one biosensor is arranged to detect the second signal generated from the target 105. For example, the bracelet broadly detects, using the biosignal sensor 120, the second signal such as the bio-electric/magnetic signal, the bio-impedance signal, and the bio-mechanical signal.

The bracelet may be fastened onto the target 105 through being wrapped around a skin over a wrist muscle. Here, the bracelet is fastened onto the target 105 by covering the wrist muscle with an area of the bracelet, for example, the sensing surface, in which the biosignal sensor 120 is arranged. The biosignal sensor 120 collects, using the at least one biosensor, the second signal throughout an entire area of the wrist muscle as the target 105.

The determination controller 130 determines a validity of the second signal based on the first signal. The determination controller 130 determines whether the second signal obtained using the biosignal sensor 120 is generated from the target 105 due to an intentional motion or incidentally generated from the target 105 due to an unintentional or spontaneous motion made irrespective of an intention of a user for operation.

The determination controller 130 determines the second signal to be valid when a magnitude of the first signal satisfies a first threshold.

Here, the first threshold is a reference value used to determine the intentional motion of the target 105, or is determined to be an average value of magnitudes of first signals obtained when the user moves a muscle for an actual operation.

The determination controller 130 determines the first threshold based on a magnitude of the second signal to be obtained. The determination controller 130 determines the first threshold for an operation involving a large motion of the target 105 to be greater in proportion to the magnitude of the second signal. Accordingly, the second signal generated by the intentional motion is not ignored due to determing a small threshold.

For example, the determination controller 130 determines the first threshold for a quick display shift operation of a display apparatus for which a relatively greater second signal is generated to be greater than the first threshold for an ON/OFF operation of an illumination apparatus for which a relatively smaller second signal is generated.

In a case of determining a validity of a second signal, the determination controller 130 identifies an interval during which the magnitude of the first signal is less than or equal to the first threshold and determine a signal, among the second signals, obtained in the identified interval to be a valid second signal. The determination controller 130 determines, to be valid, the second signal obtained in the interval during which the magnitude of the first signal is less than or equal to the first threshold and a small motion of the target 105 is performed.

When the magnitude of the second signal does not satisfy a second threshold, the determination controller 130 determines the second signal to be invalid, irrespective of the first signal. Here, the second threshold is a reference value used to determine a second signal which is valuable as information, or is determined to be an averaged minimum value when the user moves a muscle for an actual operation.

When the magnitude of the second signal is less than or equal to the determined second threshold, the determination controller 130 determines whether the target 105 moves intentionally or unintentionally to be indefinite and invalidate the second signal. Conversely, when the magnitude of the second signal is greater than the determined second threshold, the determination controller 130 further determines whether the magnitude of the first signal satisfies the first threshold and determine the validity of the second signal.

Based on the determination, the determination controller 130 operates in an active mode when the second signal is determined to be valid and allows an external device to be controlled based on the second signal. The external device is a general term for devices controlled by a command signal, such as a mobile device, a television (TV) screen, a digital versatile disc (DVD) player, a radio, a heating thermostat, a vehicle door, and the like. The command signal is generated in association with the valid second signal of the wearable biosignal interface 100.

When the magnitude of the first signal is less than or equal to the first threshold and the magnitude of the second signal is greater than the second threshold, the determination controller 130 shifts a mode of the wearable biosignal interface 100 to the active mode and allows the external device to be controlled based on the second signal and thus, improves the accuracy in determining an intention of a user for operation.

In a case of controlling the external device after the shift to the active mode, the wearable biosignal interface 100 transmits the command signal based on the valid second signal to an appliance, for example, a TV, an air conditioner, a computer, a refrigerator, and the like, or a living environment device, for example, a light, a curtain, a window, a door lock device, and the like, and enable the external device to perform a control operation, for example, an ON/OFF operation and an open/close operation, requested by the command signal.

The wearable biosignal interface 100 uses the valid second signal as a signal of a remote controller of a vehicle to start the vehicle or uses the valid second signal as an ID signal to make a payment.

The wearable biosignal interface 100 uses the valid second signal for an interaction control with a computer, or to edits a file stored on the computer or moves a page for a presentation.

The wearable biosignal interface 100 invalidates a biosignal generated by the unintentional motion of the target 105 and thus, optimally avoids a control error for the external device.

The wearable biosignal interface 100 identifies a biosignal generated only when the target 105 is at a certain level of a stabilized status and uses the biosignal to control the external device and thus, supports a precise determination of an intention of the user for operation.

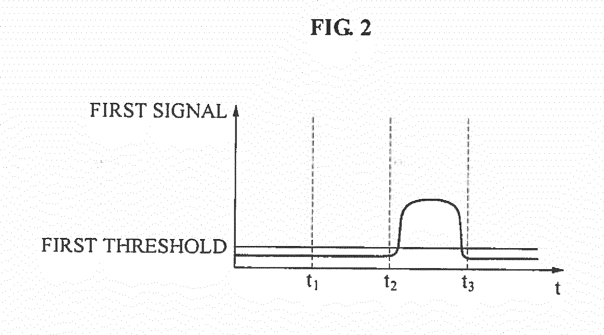

FIGS. 2 through 5 are diagrams illustrating examples of determining a valid second signal based on a first signal and a second signal according to an embodiment of present invention.

FIG. 2 is a diagram illustrating an example of a first signal obtained using a motion sensor 110 of FIG. 1 of a wearable biosignal interface 100 of FIG. 1. When a target 105 of FIG. 1 performs a rapid motion, for example, a motion of flexing an arm, in a interval from t₂ to t₃, the motion sensor 110 obtains the first signal having a magnitude measured to be great in the interval from t₂ to t₃. The first signal obtained in the interval from t₂ to t₃ is greater than a first threshold used as a reference value to determine an intentional motion of the target 105.

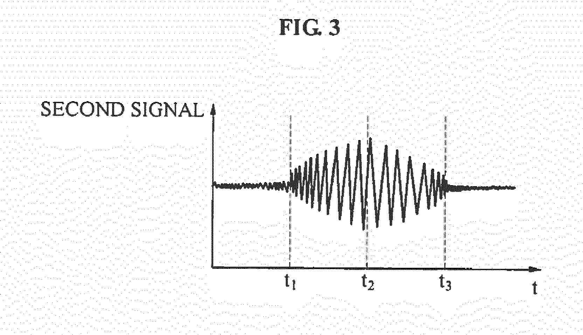

FIG. 3 is a diagram illustrating an example of a second signal obtained by a biosignal sensor 120 of FIG. 1 of a wearable biosignal interface 100 of FIG. 1. As shown in FIG. 3, the obtained second signal is in a form of an analog sine waveform and has a greater waveform in a interval from t₁ to t₃ during which muscles of an arm are tensely flexed.

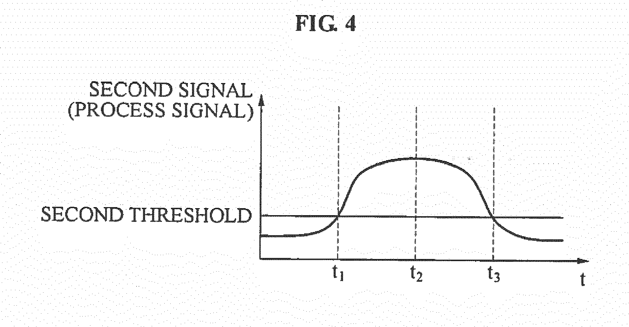

FIG. 4 is a graph illustrating an example in which a determination controller 130 of FIG. 1 of a wearable biosignal interface 100 of FIG. 1 processes magnitudes of the obtained second signals and compares the magnitudes. The determination controller 130 plots, based on the second signal of FIG. 3, a parabola in which an inclination increases drastically after a point t₁ at which a waveform becomes larger and the inclination decreases drastically after a point t₃ at which the waveform becomes smaller.

The second signal in the interval from t₁ to t₃ is obtained when satisfying a second threshold used as a reference value to determine a second signal which is valuable as information.

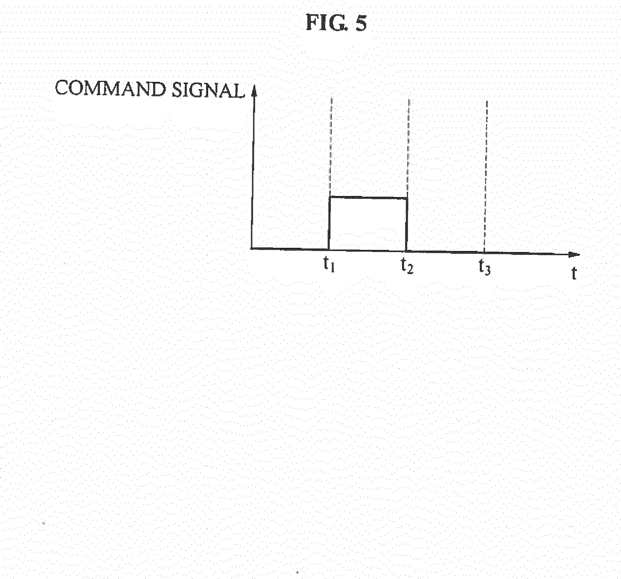

FIG. 5 illustrates an example of determining, by a determination controller 130 of FIG. 5, a validity of a second signal based on a first signal and a second signal.

The determination controller 130 invalidates the second signal obtained in the interval from t₂ to t₃ in which the first signal exceeding the first threshold is obtained. Also, the determination controller 130 invalidates the second signal obtained during an interval before t₁ and after t₃ during which the second signal less than or equal to the second threshold is obtained.

As shown in FIG. 5, the determination controller 130 determines the second signal obtained in of the inteval from t₁ to t₂ to be a valid signal and enables a command signal to be generated to control an external device during the interval.

The wearable biosignal interface 100 operates, using the second signal, the external device, for example, a mobile device, a TV screen, a DVD, a radio, a heating thermostat, and a vehicle door, although the wearable biosignal interface 100 allows the operation of the external device, using the second signal, only when the magnitude of the first signal decreases to be less than or equal to the first threshold due to a small motion performed by a user so as to improve accuracy of the wearable biosignal interface 100 in determining an intention of a user for operation.

Also, the wearable biosignal interface 100 allows the operation of the external device when the magnitude of the second signal satisfies the second threshold and accordingly the second signal which is valuable as information.

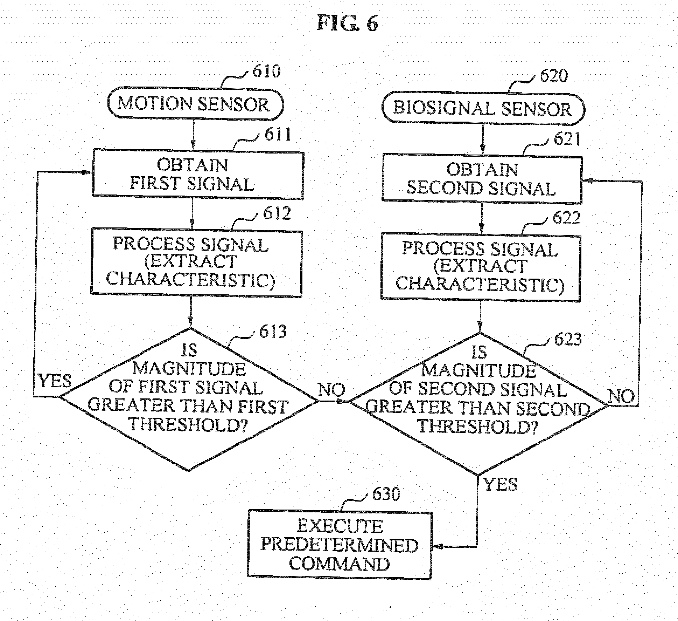

FIG. 6 is a diagram illustrating an example of performing a command given to an external device, using a magnitude of a first signal and a magnitude of a second signal.

As shown in FIG. 6, in 611, a motion sensor 610 of a wearable biosignal interface detects a motion of a target and obtain a first signal. For example, the motion sensor 610 obtains a first signal associated with flexing an arm as the target.

In 612, the wearable biosignal interface processes the first signal obtained by the motion sensor 610 and extract a characteristic. For example, wearable biosignal interface extracts the magnitude of the first signal corresponding to a characteristic associated with a speed at which an arm is flexed.

In 613, the wearable biosignal interface determines whether the magnitude of the first signal is greater than a first threshold. For example, the wearable biosignal interface determines a magnitude of a first signal obtained when a user moves a muscle to actually operate an external device to be the first threshold, and compares the first threshold to magnitudes of the obtained first signals.

Also, in 613, the wearable biosignal interface identifies an interval during which the magnitude of the first signal is less than or equal to the first threshold.

When the magnitude of the first signal is greater than the first threshold, for example, in a 'Yes' direction from 613, the wearable biosignal interface ignores the second signal obtained in the same interval during which the first signal is obtained. Also, the wearable biosignal interface returns to 611 and re-obtains the first signal.

Conversely, when the magnitude of the first signal is less than or equal to the first threshold, for example, in a 'No' direction of 613, the wearable biosignal interface proceeds to 623, as descibed later, and determines whether the second signal is valid based on a result of comparing the magnitude of the second signal obtained in the same interval during which the first signal is obtained to the second threshold.

In 621, a biosignal sensor 620 of the wearable biosignal interface obtains a second signal from the target. For example, the biosignal sensor 620 obtains the second signal when a tensely flexed arm is the target.

In 622, the wearable biosignal interface processes the second signal obtained by the biosignal sensor 620 and extracts a characteristic. For example, the wearable biosignal interface extracts a magnitude of the second signal corresponding to a characteristic associated with tension in the tensley flexed arm.

In 623, the wearable biosignal interface determines whether the magnitude of the second signal is greater than a second threshold. For example, the wearable biosignal interface determines a reference value of the magnitude of the second signal which is valuable as information to be the second threshold and compares the second threshold to magnitudes of the obtained second signals. In 623, the wearable biosignal interface determines a signal obtained in the identified interval during which the magnitude of the first signal is less than or equal to the first threshold to be a valid second signal.

When the magnitude of the second signal is less than or equal to the second threshold, for example, a 'No' direction of 623, the wearable biosignal interface ignores the obtained second signal, irrespective of a result of 613. Also, the wearable biosignal interface returns to 621 and re-obtains the second signal.

Conversely, when the magnitude of the second signal is greater than the second threshold, for example, in a 'Yes' direction of 623, the wearable biosignal interface ultimately determines the second signal to be valid based on a result of 613 and proceeds to 630.

Operations described in the foregoing include obtaining, by the motion sensor 610, a first signal in 611 and obtaining, by the biosignal sensor 620, a second signal in 621. However, it is possible to perform 321 prior to or concurrent with 311.

In 630, the wearable biosignal interface executes a command based on the second signal determined to be valid. In 630, the wearable biosignal interface operates in an active mode and allows an external device to be controlled based on the second signal. The wearable biosignal interface operates, using the valid second signal, the external device, for example, a mobile device, a TV screen, a DVD player, a radio, a heating thermostat, and a vehicle door.

Hereinafter, operations of a wearable biosignal interface 100 according to an embodiment of present invention will be described.

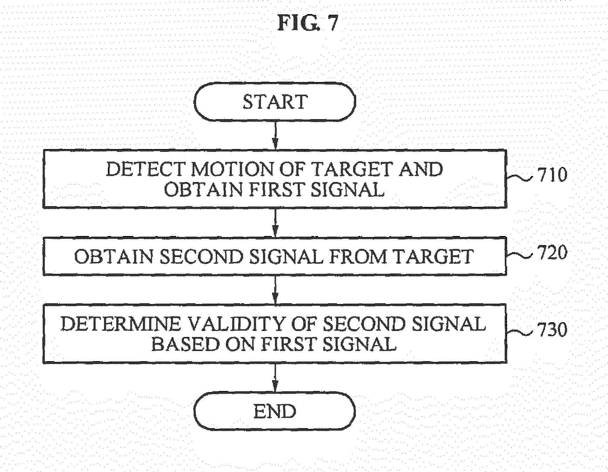

FIG. 7 is a flowchart illustrating an operation method of the wearable biosignal interface 100 according to an embodiment of the present invention.

In 710, the wearable biosignal interface 100 detects a motion of a target 105 of

FIG. 1 and obtains a first signal. Here, the first signal may be an acceleration signal, a rotation signal, a bearing signal, and a coordinate signal, associated with a velocity variance based on a motion status of the target 105. In 710, the wearable biosignal interface 100 monitors, using a motion sensor 110 of FIG. 1, the motion of the target 105 and receives the first signal to perceive a movement involved with the motion. For example, the wearable biosignal interface 100 identifies a gesture performed by the target 105 when a biosignal is obtained, and obtains the first signal associated with the identified gesture.

For example, the motion sensor 110 may include an acceleration sensor. In this case, the motion sensor 110 converts an acceleration signal obtained as the first signal into a velocity component, using an integral calculus. The wearable biosignal interface 100 numerically indicates a magnitude of a motion of the target.

For example, when the target 105 is an arm which is flexed, the wearable biosignal interface 100 obtains the first signal associated with an "arm motion" based on the flexing. Also, the wearable biosignal interface 100 measures the magnitude of the obtained first signal and measures a velocity magnitude of the "arm motion."

The motion sensor 110 may include a gyrosensor. In this case, the wearable biosignal interface 100 measures, using the gyrosensor, a rotation signal corresponding to the first signal.

When the target 105 is an arm performing a large rotation, the wearable biosignal interface 100 obtains the first signal associated with an "arm rotation" based on the rotating. Also, the wearable biosignal interface 100 measures the magnitude of the obtained first signal and measure an anglular velocity magnitude of the "arm rotation."

The motion sensor 110 may include a geomagnetic sensor or a GPS sensor. In this case, the motion sensor 110 measures, using the geomagnetic sensor or the GPS sensor, a bearing signal or a coordinate signal corresponding to the first signal.

According to an embodiment, the motion sensor 110 is fixed to a form of a bracelet fastened onto the target 105, along with a biosignal sensor 120.

The wearable biosignal interface 100 is in contact with the target 105 and obtains a second signal from the target 105. Here, the second signal is a biosignal associated with biological information, for example, muscle contraction and muscle relaxation, occurring in the target 105. The second signal may be a bio-electric/magnetic signal, a bio-impedance signal, and a bio-mechanical signal generated in association with a muscle.

In 720, the wearable biosignal interface 100 is in contact with the target 105 through the biosignal sensor 120 and obtains the second signal generated by at least one of muscle contraction and muscle relaxation. When the target 105 is a wrist, the wearable biosignal interface 100 detects the second signal generated by muscle contraction or muscle relaxation of the wrist.

Here, the target 105 may be a wrist, a forearm, a face, a neck, and the like of a living object, in which a muscle generating the second signal is positioned.

The biosignal sensor 120 includes at least one biosensor detecting the second signal from a muscle of the target 105 and monitors, using the at least one biosensor, contraction or relaxation of the muscle. The biosensor may be a photo sensor, a piezoelelctric sensor, a force sensor, an EMG sensor, and the like.

For example, the biosignal sensor 120 may include the photo sensor. In this case, the wearable biosignal interface 100 obtains, using the photo sensor, the second signal associated with a status of the target 105, based on light scattering.

The biosignal sensor 120 may include the piezoelelctric sensor or the force sensor. In this case, the wearable biosignal interface 100 obtains, using the piezoelelctric sensor or the force sensor, the second signal associated with an extension and contraction status of the target 105.

The biosignal sensor 120 may include the EMG sensor. In this case, the wearable biosignal interface 100 obtains, using the EMG sensor, the second signal associated with muscle contraction or relaxation of the target 105.

The biosignal sensor 120 is fixed to the bracelet fastened onto the target 105, along with the motion sensor 110. Here, a sensing surface of the biosignal sensor 120 is fixed to the bracelet to be in a direct contact with the target 105. On the sensing surface, the at least one biosensor is arranged to detect the second signal generated from the target 105. For example, the bracelet detects, using the biosignal sensor 120, various forms of the second signal such as a bio-electric/magnetic signal, a bio-impedance signal, and a bio-mechanical signal associated with the target 105.

The bracelet is fastened onto the target 105, wrapping around a skin over a wrist muscle. Here, the bracelet is fastened onto the target 105 by covering the wrist muscle with an area, for example, the sensing surface, of the bracelet in which the biosignal sensor 120 is disposed. The biosignal sensor 120 collects, using the at least one biosensor, the second signal throughout an entire area of the wrist muscle as the target 105.

In 730, the wearable biosignal interface 100 determines a validity of the second signal based on the first signal. In 730, the wearable biosignal interface 100 determines whether the second signal is generated by an intentional motion of the target 105 or incidentally generated by an unintentional or spontaneous motion of the target 105.

The wearable biosignal interface 100 determines the second signal to be valid when the magnitude of the first signal satisfies a first threshold.

Here, the first threshold is a reference value used to determine the intentional motion of the target 105 or is determined to be an average value of magnitudes of the first signals obtained when a user moves a muscle for an actual operation.

The wearable biosignal interface 100 determines the first threshold based on a magnitude of the second signal to be obtained. The wearable biosignal interface 100 determines the first threshold for operation involving a large motion of the target 105 to be greater in proportion to the magnitude of the second signal and thus, the second signal generated by the intentional motion of the target 105 is not ignored due to a threshold determined to be small.

The wearable biosignal interface 100 determines the first threshold for a quick display shift operation of a display apparatus for which a relatively greater second signal is generated to be greater than the first threshold for an ON/OFF operation of an illumination apparatus for which a relatively smaller second signal is generated.

In a case of determining a validity of the second signal, the wearable biosignal interface 100 identifies interval during which the magnitude of the first signal is less than or equal to the first threshold and determines a signal, among the second signals, obtained in the identified interval to be a valid second signal. The wearable biosignal interface 100 determines the second signal obtained in the interval during which the magnitude of the first signal is less than or equal to the first threshold and a small motion of the target 105 is determined to be valid.

The wearable biosignal interface 100 determines the second signal to be invalid, irrespective of the first signal, when the magnitude of the obtained second signal does not satisfy a second threshold. Here, the second threshold is a reference value used to determine the second signal which is valuable as information, or is determined to be an averaged minimum value when a user moves a muscle for an actual operation.

When the magnitude of the obtained second signal is less than or equal to the second threshold, the wearable biosignal interface 100 determines whether the target 105 moves intentionally for operation to be indefinite and accordingly, invalidates the second signal. Conversely, when the magnitude of the second signal is greater than the second threshold, the wearable biosignal interface 100 further determines whether the magnitude of the first signal satisfies the first threshold and determine the validity of the second signal.

Based on a result of the determination, when the second signal is determined to be valid, the wearable biosignal interface 100 operates in an active mode and allows an external device to be controlled by the second signal. The external device is a general term for devices controlled by a command signal, such as a mobile device, a TV screen, a DVD player, a radio, a heating thermostat, a vehicle door, and the like. The command signal is generated, using the wearable biosignal interface 100, based on the valid second signal.

For example, when the magnitude of the first signal is less than or equal to the first threshold and the magnitude of the second signal exceeds the second threshold, the wearable biosignal interface 100 shifts a mode of the wearable biosignal interface 100 to the active mode and allows the external device to be controlled based on the second signal and thus, improves the accuracy in determining an intention of a user for operation.

In a case of controlling the external device after the shift to the active mode, the wearable biosignal interface 100 transmits the command signal based on the valid second signal to an appliance, for example, a TV, an air conditioner, a computer, a refrigerator, and the like, or a living environment device, for example, a light, a curtain, a window, a door lock device, and the like, and enable the external device to perform a control operation, for example, an ON/OFF operation and an open/close operation, requested by the command signal.

The wearable biosignal interface 100 uses the valid second signal as a signal of a remote controller of a vehicle to start the vehicle, or as an identification (ID) signal to make a payment.

The wearable biosignal interface 100 uses the valid second signal for an interaction control with a computer, or to edit a file stored on the computer or move a page for a presentation.

The wearable biosignal interface 100 invalidates a biosignal generated by the unintentional or spontaneous motion of the target 105 and thus, optimally avoids a control error for the external device.

The wearable biosignal interface 100 identifies a biosignal exclusively generated when the target 105 is in a certain level of a stabilized status and uses the biosignal to control the external device and thus, supports a precise determination of an intention of a user for operation.

The method described herein according to example embodiments may be recorded in non-transitory computer-readable media including program instructions to implement various operations embodied by a computer. The media may also include, alone or in combination with the program instructions, data files, data structures, and the like. Examples of non-transitory computer-readable media include magnetic media such as hard disks, floppy disks, and magnetic tape; optical media such as CD ROM discs and DVDs; magneto-optical media such as floptical discs; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described example embodiments, or vice versa.

While this disclosure includes specific examples, it will be apparent to one of ordinary skill in the art that various changes in form and details may be made in these examples without departing from the scope of the claims.

The foregoing description describes several embodiments, examples and/or aspects. However the described embodiments, examples and/or aspects are not to be regarded as necessarily defining the invention, unless they fall within the scope of the appended claims. Therefore the scope of protection of the present invention is solely defined by the appended claims.

## Claims

1. An operation method of a wearable biosignal interface (100), the method comprising:
using a motion sensor (110) to obtain a first signal based on a motion of a user;
using a biosignal sensor (120) disposed on the user to obtain a second signal; and
determining a validity of the second signal;
**the method** being **characterized in that**
the validity of the second signal is determined to be valid, when a magnitude of the second signal is above a second threshold, while a magnitude of the first signal is less than or equal to a first threshold,
wherein a value of the first threshold is determined based on a magnitude of the second signal; and
controlling an external device based on the second signal, in response to the second signal being determined to be valid.

2. The method of claim 1, wherein the second signal includes biological information obtained by at least one biosensor in contact with the user.

3. A wearable biosignal interface (100) comprising:
a motion sensor (110) configured to obtain a first signal based on a motion of a user;
a biosignal sensor (120) disposed on the user and configured to obtain a second signal from the user; and
the wearable bio signal interface (100) being **characterized in that**
a determination controller (130) is configured to determine a validity of the second signal to be valid, when a magnitude of the second signal is above a second threshold, while a magnitude of the first signal is less than or equal to a first threshold,
wherein a value of the first threshold is determined based on a magnitude of the second signal; and
the wearable biosignal interface is configured to control an external device based on the second signal, in response to the second signal being determined to be valid.

## Patentansprüche

1. Ein Verfahren zum Betreiben einer tragbaren Biosignalschnittstelle (100), wobei das Verfahren umfasst:
Verwenden eines Bewegungssensors (110), um ein erstes Signal basierend auf einer Bewegung eines Benutzers zu erhalten;
Verwenden eines Biosignalsensors (120), der an dem Benutzer angeordnet ist, um ein zweites Signal zu erhalten; und
Bestimmen einer Gültigkeit des zweiten Signals;
**das Verfahren dadurch gekennzeichnet ist, dass**
die Gültigkeit des zweiten Signals als gültig bestimmt wird, wenn eine Größe des zweiten Signals über einem zweiten Schwellenwert liegt, während eine Größe des ersten Signals kleiner als oder gleich einem ersten Schwellenwert ist,
wobei ein Wert des ersten Schwellenwertes basierend auf einer Größe des zweiten Signals bestimmt wird; und
Steuern einer externen Vorrichtung auf Grundlage des zweiten Signals als Reaktion darauf, dass das zweite Signal als gültig bestimmt wurde.

2. Das Verfahren nach Anspruch 1, wobei das zweite Signal biologische Informationen enthält, die von mindestens einem Biosensor in Kontakt mit dem Benutzer erhalten werden.

3. Eine tragbare Biosignalschnittstelle (100), umfassend:
einen Bewegungssensor (110), der konfiguriert ist, um ein erstes Signal basierend auf einer Bewegung eines Benutzers zu erhalten;
einen Biosignalsensor (120), der an dem Benutzer angeordnet und konfiguriert ist, um ein zweites Signal von dem Benutzer zu erhalten; und
**die tragbare Biosignalschnittstelle (100) dadurch gekennzeichnet ist, dass**
eine Bestimmungssteuerung (130) konfiguriert ist, um eine Gültigkeit des zweiten Signals als gültig zu bestimmen, wenn eine Größe des zweiten Signals über einem zweiten Schwellenwert liegt, während eine Größe des ersten Signals kleiner oder gleich einem ersten Schwellenwert ist,
wobei ein Wert des ersten Schwellenwertes basierend auf einer Größe des zweiten Signals bestimmt wird; und
die tragbare Biosignalschnittstelle konfiguriert ist, um ein externes Gerät basierend auf dem zweiten Signal zu steuern, als Reaktion darauf, dass das zweite Signal als gültig bestimmt wird.

## Revendications

1. Procédé de fonctionnement d'une interface de biosignal vestimentaire (100), le procédé comprenant :
utilisation d'un capteur de mouvement (110) pour obtenir un premier signal fondé sur un mouvement d'un utilisateur ;
utilisation d'un capteur de biosignal (120) disposé sur l'utilisateur pour obtenir un deuxième signal ; et
détermination d'une validité du deuxième signal ;
**le procédé étant caractérisé en ce que**
la validité du deuxième signal est déterminée pour être valide, lorsqu'une amplitude du deuxième signal est supérieure à un deuxième seuil, tandis qu'une amplitude du premier signal est inférieure ou égale à un premier seuil,
dans lequel une valeur du premier seuil est déterminée selon une amplitude du signal ; et
contrôle d'un dispositif externe fondé sur le deuxième signal, en réponse au deuxième signal déterminé comme valide.

2. Le procédé de la revendication 1, dans lequel le deuxième signal inclut des informations biologiques obtenues par au moins un biocapteur en contact avec l'utilisateur.

3. Interface de biosignal vestimentaire (100) comprenant :
un capteur de mouvement (110) configuré pour obtenir un premier signal fondé sur un mouvement d'un utilisateur ;
un capteur de biosignal (120) disposé sur l'utilisateur et configuré pour obtenir un deuxième signal de l'utilisateur ; et
l'interface de biosignal vestimentaire (100) étant **caractérisée en ce que**
un contrôleur de détermination (130) est configuré pour déterminer une validité du deuxième signal à valider,
lorsqu'une amplitude du deuxième signal est supérieure à un deuxième seuil, tandis qu'une amplitude du premier signal est inférieure ou égale à un premier seuil,
dans lequel une valeur du premier seuil est déterminée selon une amplitude du deuxième signal ; et
l'interface de biosignal vestimentaire est configurée pour contrôler un dispositif externe fondé sur le deuxième signal, en réponse au deuxième signal déterminé comme valide.
